# EUROPÄISCHE PATENTSCHRIFT

(11) **EP 1 721 579 B1**
(45) Veröffentlichungstag und Bekanntmachung des Hinweises auf die Patenterteilung: **08.07.2009**
(21) Anmeldenummer: 06450032.5
(22) Anmeldetag: 09.03.2006
(51) Int. Cl.: A61B 17/435

(54) **Katheter zum uterinen Embryonentransfer**
Embryo transfer catheter
Cathéter pour transfert d'embryon

(30) Priorität: 10.05.2005 AT 7972005
(43) Veröffentlichungstag der Anmeldung: 15.11.2006
(73) Patentinhaber: Steiner, Hans-Peter, 8010 Graz (AT)
(72) Erfinder: Steiner, Hans-Peter, 8010 Graz (AT)
(74) Vertreter: Babeluk, Michael

(56) Entgegenhaltungen:
- WO-A-01/21081
- US-A- 735 400
- US-A- 4 004 588

## Beschreibung

Die Erfindung betrifft eine Vorrichtung für den uterinen Embryonentransfer mit einem Führungskatheter und einem in diesem geführten Innenkatheter, wobei der Führungskatheter an seinem distalen Ende biegeweich ausgeführt ist und ein Führungselement mit einer distalen Öffnung aufweist. Weiters betrifft die Erfindung einen Führungskatheter zur Aufnahme eines Innenkatheters für den uterinen Embryonentransfer oder dgl.

Ein ganz wesentlicher Schritt, welcher über Erfolg oder Misserfolg einer In-Virto-Fertilisierung (IVF) entscheidet, ist der Embryonentransfer. In einigen Fällen werden heute die am Markt angebotenen einlumigen Kunststoffkatheter verwendet, welche aus einem flexiblen und an der Spitze möglichst runden, atraumatisch einführbaren Kunststoffrohr bestehen.

Häufiger werden zweiteilige Katheter verwendet, deren äußeres, relativ starres Plastikrohr bis zur Portio geführt wird, wobei dann der innere Katheter, welcher mit der Trägerlösung samt den Embryonen gefüllt ist, ins Innere des Uterus vorgeschoben wird. Das Ziel ist eine möglichst schmerzlose und unblutige Passage des Zervikalkanals, wobei man die Embryonen in etwa ein Zentimeter Entfernung vom Uterusfundus entlässt. Vor der eigentlichen Behandlung muss der Uterus mittels eines Probe-Kunststoffkatheters oder unmittelbar während eines Embryotransfers durch eine gleichzeitige Ultraschallkontrolle vermessen werden.

In etwa 10 % der Fälle kommt es allerdings vor, dass der weiche, flexible Embryotransferkatheter in Folge eines zu engen, gewundenen oder zerklüfteten Zervikalkanals diesen nicht überwinden kann und die Behandlung abgebrochen werden muss. In diesem Fall bieten die Hersteller herkömmlicher zweiteiliger Katheter einen in den Führungskatheter einführbaren Mandrin an (ein mit Kunststoff überzogener Draht), dessen Krümmung der Arzt in Abhängigkeit der von Patientin zu Patientin unterschiedlichen Uterusanatomie verformen bzw. entsprechend biegen kann, um so die Richtung des Zervikalkanals und dessen Abwinkelung nachvollziehen zu können.

Falls nun der Innenkatheter auf einen nicht überwindbaren Widerstand stößt, muss dieser bei herkömmlichen Führungskathetern entfernt und ein Metallmandrin eingeführt werden. Sobald mit diesem der Widerstand überwunden ist, wird der Mandrin aus dem Führungskatheter entfernt und wiederum der Innenkatheter eingeschoben. Der Vorgang ist allerdings für den Reproduktionsmediziner und die Patientin äußerst stressreich und führt häufig zu gefürchteten Blutungen, die die Chance einer erfolgreichen Implantation der Embryonen und somit einer Schwangerschaft stark reduzieren.

In diesem Zusammenhang ist aus der EP 0 526 669 A1 ein Instrumentensatz für den uterinen Embryonentransfer bekannt geworden, welcher einen äußeren Führungskatheter und einen darin geführten Innenkatheter aufweist. Der Führungskatheter besteht aus flexiblem Teflon und schließt an seinem distalen Ende mit einer Kugel mit einer distalen Öffnung ab. Der Außendurchmesser der Kugel beträgt das 3,5-fache des Innendurchmessers des Führungskatheters an seiner distalen Öffnung. An seinem proximalen Ende weist der Führungskatheter eine Kupplungshülse auf, die zugleich ein Griffstück für die Handhabung bildet.

Der in den Führungskatheter einführbare Innenkatheter weist einen Schlauch aus poliertem Kunststoff auf, der im proximalen Bereich von einem Verstärkungsrohr aus Edelstahl umschlossen ist. Sowohl der Führungskatheter als auch der Innenkatheter weisen in deren distalen Bereich eine leichte Krümmung auf, die das Einführen des Instrumentensatzes erleichtern soll.

In der US 4,004,588 A wird ein uteriner Embryotransferkatheter für die Tiermedizin beschrieben, welcher im Wesentlichen vierteilig aufgebaut ist. Die Vorrichtung besteht aus einem Außenkatheter mit einer aufblasbaren Manschette und zwei seitlichen Fluidanschlüssen, einem Innenkatheter, der in den Außenkatheter eingeschoben wird, einer auf den Innenkatheter aufschraubbaren, flexiblen Leitung, sowie aus einem Stahlmandrin, der in die flexible Leitung eingeführt wird. Die flexible Leitung besteht bis auf ein konisches Anschlusselement zum Innenkatheter zur Gänze aus einer Spiralfeder aus rostfreiem Stahl, die mit einem sterilisierbaren Kunststoffüberzug versehen ist. Um die flexible Leitung in den Uterus einführen zu können, ist der Stahlmandrin vorgesehen, der die frontseitige Öffnung eines Führungselementes der flexiblen Leitung beim Einführen in den Uterus verschließt.

Aus der WO 01/21081 A1 ist ein Embryotransferkatheter bekannt, bei welchem in mehreren Ausführungsvarianten unterschiedliche Ultraschallreflektoren gezeigt werden, die die Handhabung des Katheters unter Ultraschallanwendung erleichtern.

Aus der EP 0 131 166 A1 ist eine Vorrichtung für den uterinen Embryonentransfer bekannt, welche einen doppellumigen Katheter aufweist, der aus zwei konzentrisch angeordneten Kunststoffröhrchen besteht. Das innere Röhrchen weist am distalen Ende eine abgerundete Kappe auf und dahinter Sprühöffnungen für eine durch das innere Lumen einbringbare Lösung. Die Sprühöffnungen sind durch einen Käfig aus einer spiraligen Struktur geschützt, welcher sich von der abgerundeten Kappe bis zur Öffnung des äußeren Kunststoffröhrchens erstreckt. Das äußere Lumen steht mit einem Behälter der Vorrichtung in Verbindung, in welchem Unterdruck angelegt werden kann. Der doppellumige Katheter weist proximal eine Metallhülle auf, die etwa 2 cm bis 2,5 cm in den Zervikalkanal vorgeschoben wird. In vielen Fällen ist es allerdings schwierig die starre Metallhülle atraumatisch in den Gebärmutterhalskanal einzubringen.

Aufgabe der Erfindung ist es, ausgehend von bekannten Vorrichtungen für den uterinen Embryonentransfer auf der Basis zweiteiliger Katheter, die Technik des Embryotransfers zu verbessern, so dass der Embryotransfer wesentlich vereinfacht und auch bei schwieriger Uterusanatomie mit einer möglichst hohen Erfolgsquote durchgeführt werden kann.

Erfindungsgemäß wird dies dadurch erreicht, dass der Führungskatheter aus einem Metallrohr besteht, an dessen proximalem Ende ein Spritzenadapter und an dessen distalem Ende im Wesentlichen stufenlos eine das Führungselement tragende Spiralfeder befestigt ist. Die Flexibilität der Spirale ermöglicht eine spontane Anpassung und somit eine atraumatische und blutungsfreie Überwindung aller Hindernisse (wie Krümmungen, Myomvorwölbungen im Bereiche des Zervikalkanals, etc.) Dadurch findet der Katheter auch bei geringer Druckanwendung den Weg durch den Zervikalkanal. Die metallische Ausführung des Führungskatheters ermöglicht während des gesamten Embryotransfers eine deutliche Darstellung der Position des Katheters mittels Ultraschall.

Erfindungsgemäß ist das Metallrohr in einer ersten Ausführungsvariante zumindest in seinem distalen Bereich, bevorzugt im gesamten Bereich, verformbar ausgeführt, so dass dessen Krümmung vor der Anwendung vom Arzt an die jeweilige Uterusanatomie angepasst werden kann. Dies gilt sowohl für die Lage der Krümmung, den Krümmungsradius und auch den durch die Krümmung eingestellten Winkel der distalen Katheterspitze im Bezug auf das proximale Ende des Führungskatheters.

Erfindungsgemäß kann das Metallrohr aus einem nahtlosen, weichgeglühten Edelstahlrohr bestehen, so dass dieses einerseits durch den Arzt leicht verformbar ist und andererseits die eingeprägte Form bzw. Krümmung während der Behandlung beibehält. Um den Führungskatheter mit dem Innenkatheter möglichst atraumatisch einführen zu können, ist das Führungselement am distalen Ende des Metallrohrs beispielsweise kugelförmig oder im Längsschnitt elliptisch ausgeführt, wodurch der Katheter optimal den Windungen des Zervikalkanals folgt.

Gemäß einer besonders bevorzugten, zweiten Ausführungsvariante weist die Spiralfeder im Bereich zwischen dem Metallrohr und dem Führungselement entlang deren Längsachse eine eingeprägte Krümmung auf, wobei zur Herstellung der Krümmung vorzugsweise mittels Lasertechnologie hergestellte Schweißpunkte dienen, die einzelne Paare benachbarter Windungen der Spiralfeder verbinden. Da nur einzelne benachbarte Windungen der Spiralfeder entlang einer Mantellinie parallel zur Achse der Feder miteinander verbunden sind, bleibt diese weiter beweglich und weist dennoch die für die Anwendung optimale Krümmung auf.

Es ist zwar möglich die Spiralfeder aus Kunststoff herzustellen und durch Kleben am Metallrohr zu befestigen, besonders vorteilhaft ist es allerdings, wenn die Spiralfeder aus Metall, vorzugsweise aus Edelstahl gefertigt ist, so dass die Spiralfeder erfindungsgemäß einerseits mit dem distalen Ende des Metallrohrs sowie andererseits mit dem Führungselement mittels Lasertechnologie verschweißt werden kann. Es entsteht dadurch ein am distalen Ende äußerst flexibler und im Bereich des Metallrohrs nach individuellen Erfordernissen verformbarer Führungskatheter, der wiederverwendbar, leicht zu sterilisieren und im Ultraschall gut kontrollierbar ist.

Die Erfindung bezieht sich nicht nur auf Systeme bestehend aus einem Führungskatheter und einem Innenkatheter, sondern auch auf einen separaten Führungskatheter, der aus einem Metallrohr besteht und an dessen distalem Ende eine das Führungselement tragende Spiralfeder, vorzugsweise aus Edelstahl, befestigt ist. Der Führungskatheter ist im Hinblick auf Länge und Innendurchmesser auf die am Markt erhältlichen Innenkatheter abgestimmt, so dass sich auch Kostenvorteile im Hinblick auf Einmal-Systeme ergeben, bei welchen das gesamte Set bestehend aus Führungskatheter und Innenkatheter nach einer Anwendung verworfen wird.

Der erfindungsgemäße Führungskatheter kann auch zum Einführen optischer Geräte und Instrumente für eine Gebärmutterspiegelung, sowie zum Einführen entsprechender Geräte zur Entnahme von Gewebs- und Zellproben aus der Gebärmutter verwendet werden.

Dadurch, dass der aus Metall bestehende Führungskatheter im Ultraschall besonders gut sichtbar ist, muss der Innenkatheter - ausgehend von der distalen Endposition des Führungskatheters - lediglich im vorgegebenen Maß an die gewünschte Position im Uterus vorgeschoben werden.

Die Erfindung wird im Folgenden anhand von Zeichnungen näher erläutert. Es zeigen:
- Fig. 1: eine erfindungsgemäße Vorrichtung für den uterinen Embryonentransfer mit einem Führungskatheter und einem in diesem geführten Innenkatheter;
- Fig. 2: den erfindungsgemäßen Führungskatheter der Vorrichtung gemäß Fig. 1 in einem vergrößert dargestellten Längsschnitt;
- Fig. 3: die Vorrichtung gemäß Fig. 1 in einer vom Arzt vor der Anwendung geformten, distalen Krümmung;
- Fig. 4: den distalen Teil der Vorrichtung gemäß Fig. 3 beim uterinen Embryonentransfer; sowie die
- Fig. 5: und Fig. 6 den distalen Teil einer Ausführungsvariante des erfindungsgemäßen Führungskatheter in einer Seitenansicht und in einer Draufsicht.

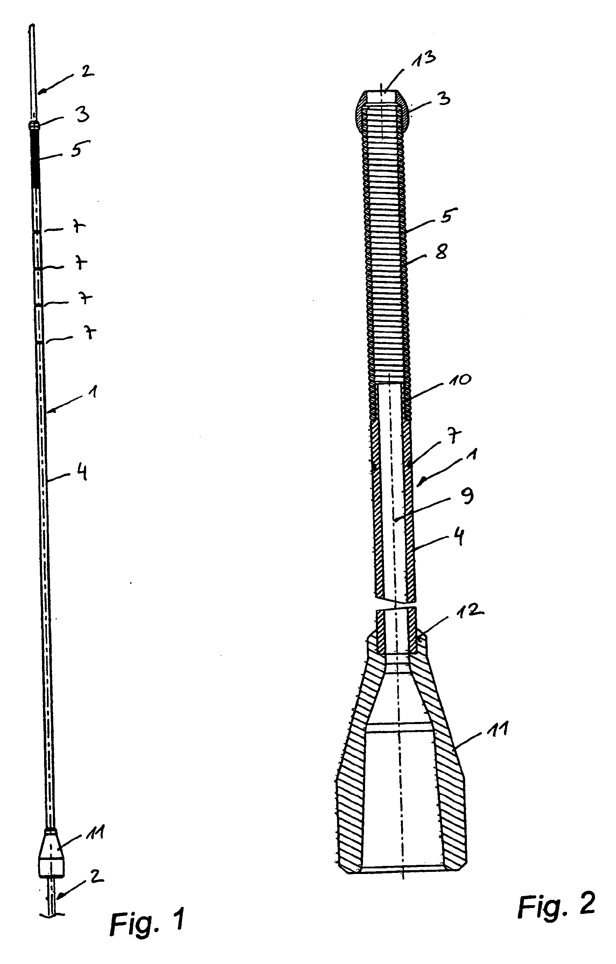

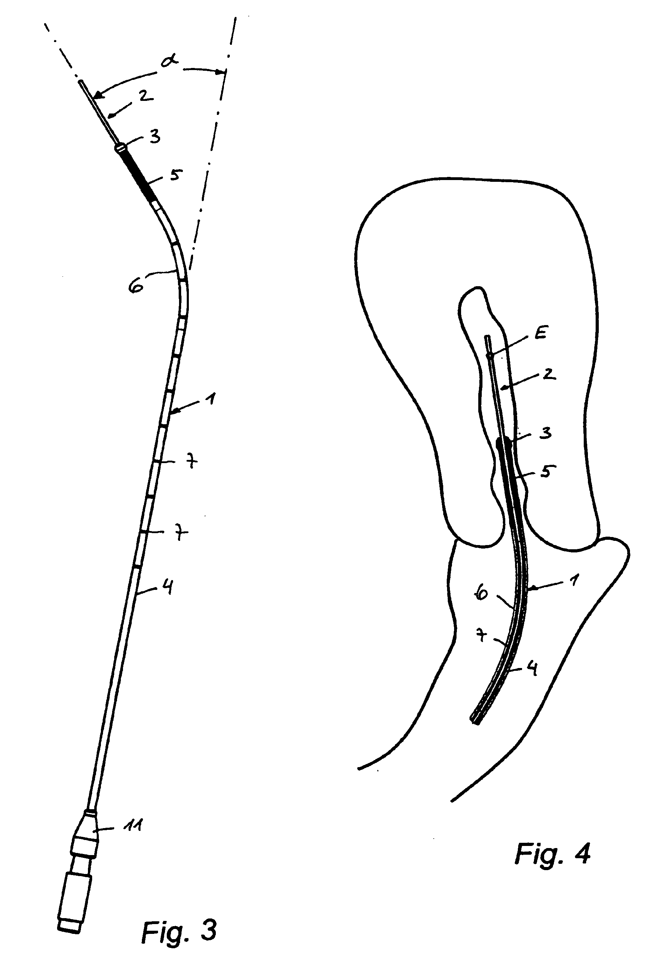

Die in Fig. 1 dargestellte Vorrichtung für den Transfer von Embryonen in den Uterus weist einen Führungskatheter 1 mit einem darin verschiebbar gehaltenen Innenkatheter 2 aus Kunststoff auf. Der Führungskatheter 1 ist zumindest an seinem distalen Ende biegeweich ausgeführt und weist ein Führungselement 3 in Form einer im Wesentlichen kugelförmigen Endkappe mit distaler Öffnung 13 auf. Der Führungskatheter 1 mit einer Gesamtlänge von ca. 20 cm besteht hauptsächlich aus einem Metallrohr 4, an dessen distalem Ende eine das Führungselement 3 tragende, kurze Spiralfeder 5 von ca. 17 mm bis 20 mm befestigt ist. Das Metallrohr 4 mit einem Außendurchmesser von z.B. 2 mm und einer Wandstärke von ca. 0,4 mm bis 0,5 mm besteht bevorzugt aus einem nahtlosen, weichgeglühten Edelstahlrohr (1.4404) und kann daher vor der Anwendung vom Arzt an die jeweilige Uterusanatomie angepasst, d.h. mit einer Krümmung 6 (siehe Fig. 3) versehen werden.

Um die Lage bzw. die Position des Führungskatheters 1 während des Einführens in den Uterus besser kontrollieren zu können, sind im distalen Bereich des Metallrohrs 4 mehrere Distanzmarkierungen 7 (z.B. im Zentimeterabstand) angeordnet. Diese ermöglichen es dem Arzt die Eindringtiefe des Katheters zusätzlich zum Ultraschall zu kontrollieren. Dies ist insbesondere für jene Fälle wichtig, bei welchen die Ultraschallkontrolle wegen extrem adipöser Patientinnen nicht oder nur erschwert möglich ist. Weiters sind die Markierungen 7 des Führungskatheters 1 auch für die Uterussondierung bzw. Uterusvermessung verwendbar.

Wie insbesondere in der Detaildarstellung gemäß Fig. 2 erkennbar, liegen die Windungen 8 der bevorzugt aus Edelstahl (1.4310) bestehenden Spiralfeder 5 in unbelastetem Zustand aneinander an, so dass ein durchgehendes Lumen 9 für den hier nicht dargestellten Innenkatheter entsteht. Die Spiralfeder 5 mit einem Außendurchmesser von ca. 2,1 mm und einer Drahtstärke von 0,3 mm ist einerseits mit dem distalen Ende des Metallrohrs 4 und andererseits mit dem Führungselement 3 mittels Lasertechnologie verschweißt. Dazu weist das Metallrohr 4 eine endseitige Abstufung 10 in der Wandstärke auf, auf welche die Spiralfeder 5 aufgeschoben und verschweißt ist, so dass an der Außenseite des Führungskatheters 1 ein im Wesentlichen stufenloser Übergang vom Metallrohr 4 zur Spiralfeder 5 erfolgt.

Am proximalen Ende des Metallrohrs 4 ist ein Spritzenadapter 11, vorzugsweise ein Luer-Anschluss, befestigt. Dieser besteht im dargestellten Beispiel aus Edelstahl (1.4310) und weist eine Ausnehmung 12 auf, in die das proximale Ende des Metallrohrs 4 mittels Lasertechnologie eingeschweißt ist.

Das Metallrohr 4 des Führungskatheters 1 wird in der Ausführungsvariante gemäß Fig. 3 vom Arzt vor der Anwendung entsprechend der Uterusanatomie verformt, so dass eine Krümmung 6 im distalen Bereich entsteht und die Katheterspitze im Bezug auf das proximale Ende des Führungskatheters um einen Winkel α von ca. 15° bis 45° angehoben wird. Bevorzugt weist der Führungskatheters 1 bereits im Lieferzustand (in der Originalverpackung) eine der durchschnittlichen Uterusanatomie entsprechende Krümmung 6 auf, so dass der behandelnde Arzt nur noch geringfügige Anpassungen vornehmen muss.

Durch die vorgeformte Krümmung 6 und die weiche Spiralfeder 5 lässt sich der Führungskatheter 1 wesentlich leichter in den Zervikalkanal (siehe Fig. 4) einführen, wobei das an der Spitze der Spiralfeder 5 angeordnete kugelförmige Führungselement 3 an der Wand des Zervikalkanals entlang gleitet. Nach Erreichen der optimalen Lage des Führungskatheters 1, welche mit Hilfe der Markierungen 7 und/oder im Ultraschallbild kontrolliert werden kann, wird der Innenkatheter 2 bis in etwa 1 cm Entfernung zum Uterusfundus vorgeschoben und der Embryo E in den Uterus entlassen.

Ein weiterer Vorteil der erfindungsgemäßen Vorrichtung besteht in der weitgehenden Vermeidung einer Kontamination des Innenkatheters 2 mit Bakterien oder Pilzen, da dieser durch die Umhüllung durch den Führungskatheter 1 mit dem äußeren Muttermund und dem Zervikalkanal nicht in Berührung kommt.

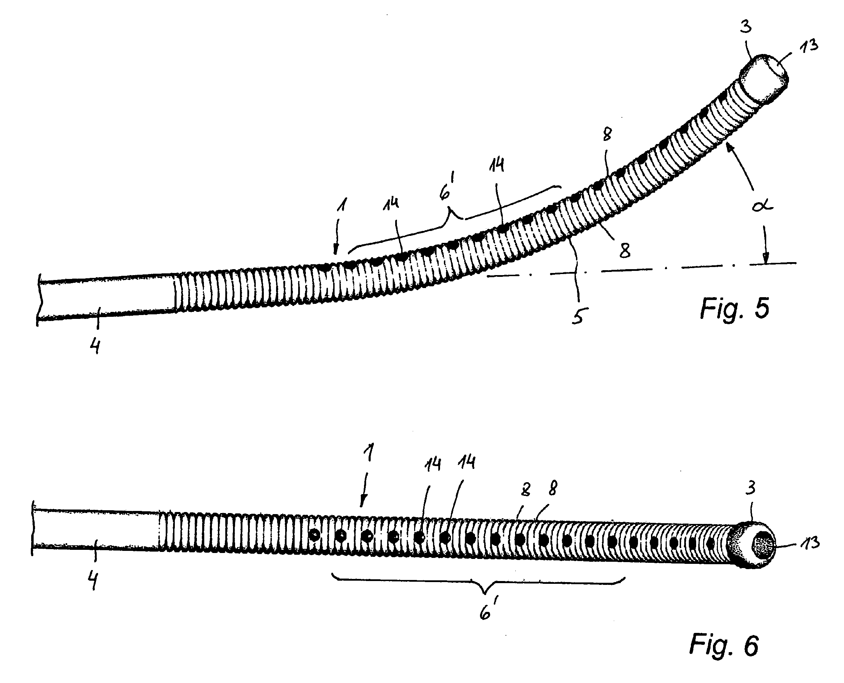

In der Ausführungsvariante gemäß Fig. 5 und Fig. 6 weist die Spiralfeder 5 im Bereich zwischen dem Metallrohr 4 und dem Führungselement 3 entlang deren Längsachse eine eingeprägte Krümmung 6' auf, wobei zur Herstellung der Krümmung 6' vorzugsweise mittels Lasertechnologie hergestellte Schweißpunkte 14 dienen, die nur einzelne Paare benachbarter Windungen 8 der Spiralfeder 5 verbinden, andere jedoch unverbunden belassen, so dass die Spiralfeder 5 weiter beweglich bleibt. Die Schweißpunkte 14 sind entlang einer Mantellinie parallel zur Achse der Feder angeordnet, so dass sich die dargestellte Krümmung 6' einstellt. Die Krümmung 6' der Spiralfeder 5 könnte auch mit Hilfe eines entsprechend gekrümmten Dorns hergestellt werden, auf welchen der Federdraht aufgewickelt wird.

Die Spiralfeder 5 bei den Ausführungsvarianten gemäß Fig. 5 und Fig. 6 weist eine Länge im Bereich von 3,5 cm bis 5,5 cm auf und weist am distalen Ende eine im Wesentlichen zylindrische Endkappe 3 auf, die im Bereich der distalen Öffnung 13 abgerundet ist. Für die Anwendung bei Patientinnen mit besonders engem äußeren Muttermund kann der Durchmesser der zylindrische Endkappe 3 im Bereich des äußeren Durchmessers der Spiralfeder 5 (ca. 2,1 mm bis 2,5 mm) liegen.

Die erfindungsgemäße Vorrichtung zeichnet sich durch eine wesentlich erhöhte Erfolgsrate bei der In-Virto-Fertilisierung aus.

## Patentansprüche

1. Vorrichtung für den uterinen Embryonentransfer mit einem Führungskatheter (1) und einem in diesem geführten Innenkatheter (2), wobei der Führungskatheter (1) an seinem distalen Ende biegeweich ausgeführt ist und ein Führungselement (3) mit einer distalen Öffnung (13) aufweist, **dadurch gekennzeichnet, dass** der Führungskatheter (1) aus einem Metallrohr (4) besteht, an dessen proximalem Ende ein Spritzenadapter (11) und an dessen distalem Ende im Wesentlichen stufenlos eine das Führungselement (3) tragende Spiralfeder (5) befestigt ist.

2. Vorrichtung nach Anspruch 1, **dadurch gekennzeichnet, dass** das Metallrohr (4) zumindest in seinem distalen Bereich, bevorzugt im ganzen Bereich, verformbar ist und dessen Krümmung (6) vor der Anwendung vom Arzt an die jeweilige Uterusanatomie anpassbar ist.

3. Vorrichtung nach Anspruch 1, **dadurch gekennzeichnet, dass** die Spiralfeder (5) im Bereich zwischen dem Metallrohr (4) und dem Führungselement (3) entlang deren Längsachse eine eingeprägte Krümmung (6') aufweist.

4. Vorrichtung nach Anspruch 3, **dadurch gekennzeichnet, dass** die Spiralfeder (5) zur Herstellung der Krümmung (6') vorzugsweise mittels Lasertechnologie hergestellte Schweißpunkte (14) aufweist, die einzelne Paare benachbarter Windungen (8) der Spiralfeder (5) verbinden.

5. Vorrichtung nach einem der Ansprüche 1 bis 4, **dadurch gekennzeichnet, dass** das Führungselement (3) als kugelförmige Endkappe mit einer distalen Öffnung (13) ausgeführt ist.

6. Vorrichtung nach einem der Ansprüche 1 bis 4, **dadurch gekennzeichnet, dass** das Führungselement (3) als zylindrische Endkappe ausgeführt ist, die im Bereich der distalen Öffnung (13) abgerundet ist.

7. Vorrichtung nach einem der Ansprüche 1 bis 6, **dadurch gekennzeichnet, dass** die Spiralfeder (5) aus Metall, vorzugsweise aus Edelstahl gefertigt ist.

8. Vorrichtung nach einem der Ansprüche 1 bis 7, **dadurch gekennzeichnet, dass** die Windungen (8) der Spiralfeder (5) in unbelastetem Zustand aneinander anliegen

9. Vorrichtung nach einem der Ansprüche 1 bis 8, **dadurch gekennzeichnet, dass** das Metallrohr (4) aus einem nahtlosen, weichgeglühten Edelstahlrohr besteht.

10. Vorrichtung nach einem der Ansprüche 1 bis 9, **dadurch gekennzeichnet, dass** die Spiralfeder (5) einerseits mit dem distalen Ende des Metallrohrs (4) und andererseits mit dem Führungselement (3) mittels Lasertechnologie verschweißt ist.

11. Vorrichtung nach einem der Ansprüche 1 bis 10, **dadurch gekennzeichnet, dass** im distalen Bereich des Metallrohrs (4) Distanzmarkierungen (7) angeordnet sind.

12. Vorrichtung nach einem der Ansprüche 1 bis 11, **dadurch gekennzeichnet, dass** der Spritzenadapter (11) ein Luer-Anschluss ist.

13. Vorrichtung nach einem der Ansprüche 1 bis 12, **dadurch gekennzeichnet, dass** der Spritzenadapter (11) aus Edelstahl gefertigt ist.

## Claims

1. Device for uterine embryo transfer comprising a guiding catheter (1) and an inner catheter (2) guided therein, said guiding catheter (1) being flexible at its distal end and featuring a guiding element (3) with a distal opening (13), **characterised in that** said guiding catheter (1) comprises a metal tube (4) at whose proximal end a syringe adapter (11) is provided and at whose distal end a helical coil (5) is attached, essentially without forming a step, which carries the guiding element (3).

2. Device according to claim 1, **characterised in that** the metal tube (4) is deformable at least in its distal region, and preferably over its total length, and that its curvature (6) is adaptable by the physician to the given uterus anatomy prior to use.

3. Device according to claim 1, **characterised in that** the helical coil (5) is provided with a permanent curvature (6') along its longitudinal axis in the region between the metal tube (4) and the guiding element (3).

4. Device according to claim 3, **characterised in that** the helical coil (5) has welding points (14), preferably realized by laser technology, which weld together individual pairs of adjacent windings (8) of the helical coil (5), thus producing the curvature (6') of the coil.

5. Device according to any of claims 1 to 4, **characterised in that** the guiding element (3) is configured as a spherical cap with a distal opening (13).

6. Device according to any of claims 1 to 4, **characterised in that** the guiding element (3) is configured as a cylindrical cap, which is rounded in the region of the distal opening (13).

7. Device according to any of claims 1 to 6, **characterised in that** the helical coil (5) is made of metal, preferably of stainless steel.

8. Device according to any of claims 1 to 7, **characterised in that** the windings (8) of the helical coil (5) lie against each other in the loadfree state.

9. Device according to any of claims 1 to 8, **characterised in that** the metal tube (4) is a seamless, annealed stainless- steel tube.

10. Device according to any of claims 1 to 9, **characterised in that** the helical coil (5) is welded by laser technology to the distal end of the metal tube (4) on the one hand and to the guiding element (3) on the other hand.

11. Device according to any of claims 1 to 10, **characterised in that** the distal region of the metal tube (4) is provided with distance markings (7).

12. Device according to any of claims 1 to 11, **characterised in that** the syringe adapter (11) is a Luer cone.

13. Device according to any of claims 1 to 12, **characterised in that** the syringe adapter (11) is made of stainless steel.

## Revendications

1. Dispositif pour le transfert embryonnaire utérin comportant un cathéter de guidage (1) et un cathéter intérieur (2) guidé dans celui-ci,
le cathéter de guidage (1) est souple à son extrémité distale et comporte un élément de guidage (3) avec une ouverture distale (13),
**caractérisé en ce que**
le cathéter de guidage (1) se compose d'un tube métallique (4) dont l'extrémité proximale comporte un adaptateur de seringue (11) et un ressort en spirale (5) portant l'élément de guidage (3) est fixé pratiquement de façon continue à son extrémité distale.

2. Dispositif selon la revendication 1,
**caractérisé en ce que**
le tube métallique (4) est déformable au moins au niveau de sa zone distale, de préférence sur toute sa zone et sa courbure (6) est adaptable avant l'application par le médecin à l'anatomie utérine respective.

3. Dispositif selon la revendication 1,
**caractérisé en ce qu'**
une courbure imposée (6') est prévue pour le ressort en spirale (5) dans la zone comprise entre le tube métallique (4) et l'élément de guidage (3) suivant son axe longitudinal.

4. Dispositif selon la revendication 3,
**caractérisé en ce que**
pour réaliser la courbure (6') le ressort en spirale (5) comporte des points de soudure (14) réalisés de préférence en technique laser, ces points de soudure reliant les paires voisines de spires (8) du ressort en spirale (5).

5. Dispositif selon l'une des revendications 1 à 4,
**caractérisé en ce que**
l'élément de guidage (3) a un capuchon d'extrémité de forme sphérique avec une ouverture distale (13).

6. Dispositif selon l'une des revendications 1 à 4,
**caractérisé en ce que**
l'élément de guidage (3) est réalisé comme capuchon d'extrémité est cylindrique et arrondi au niveau de l'ouverture distale (13).

7. Dispositif selon l'une des revendications 1 à 6,
**caractérisé en ce que**
le ressort en spirale (5) est en métal, de préférence en acier inoxydable.

8. Dispositif selon l'une des revendications 1 à 7,
**caractérisé en ce que**
les spires (8) du ressort en spirale (5) sont appliquées l'une contre l'autre lorsque le ressort n'est pas sollicité.

9. Dispositif selon l'une des revendications 1 à 8,
**caractérisé en ce que**
le tube métallique (4) est un tube en acier inoxydable, recuit, sans soudure.

10. Dispositif selon l'une des revendications 1 à 9,
**caractérisé en ce que**
le ressort en spirale (5) est soudé d'un côté à l'extrémité distale du tube métallique (4) et de l'autre côté à l'élément de guidage (3) par la technique laser.

11. Dispositif selon l'une des revendications 1 à 10,
**caractérisé par**
les repères de distance (7) prévus dans la zone distale du tube métallique (4).

12. Dispositif selon l'une des revendications 1 à 11,
**caractérisé en ce que**
l'adaptateur de seringue (11) est un raccord Luer.

13. Dispositif selon l'une des revendications 1 à 12,
**caractérisé en ce que**
l'adaptateur de seringue (11) est fabriqué en acier inoxydable.
